(19) Europäisches Patentamt
European Patent Office
Office européen des brevets

(11) **EP 1 891 912 A1**

(12) **EUROPEAN PATENT APPLICATION**

(43) Date of publication:
27.02.2008 Bulletin 2008/09

(51) Int Cl.:
*A61F 2/16* (2006.01)     *G02C 7/04* (2006.01)

(21) Application number: 07114347.3

(22) Date of filing: 14.08.2007

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK YU

(30) Priority: 23.08.2006 US 444112

(71) Applicant: Alcon Manufacturing, Ltd.
Fort Worth, TX 76134 (US)

(72) Inventors:
• Simpson, Michael
Arlington, FL 76012 (US)

• Karakelle, Mutlu
Forth Worth, TX 76132 (US)
• Vannoy, Steve
Southlake, TX 76092 (US)
• Zhang, Xiaoxiao
Fort Worth, TX 76132 (US)
• Hong, Xin
Arlington, TX 76017 (US)
• Xie, Jihong
Jacksonville, FL 32258 (US)

(74) Representative: Hanna, Peter William Derek et al
Hanna, Moore & Curley
13 Lower Lad Lane
Dublin 2 (IE)

(54) **Truncated diffractive intraocular lenses**

(57)     In one aspect, the present invention provides a method of designing a diffractive ophthalmic lens (e.g., an intraocular lens (IOL)) that includes providing an optic having an anterior refractive surface and a posterior refractive surface, wherein the optic provides a far-focus power (e.g., in a range of about 18 to about 26 Diopters (D)). A truncated diffractive structure can be disposed on at least one of the surfaces for generating a near-focus add power (e.g., in a range of about 3 D to about 4 D). And the diffractive structure can be adjusted so as to obtain a desired distribution of optical energy between the near and far foci for a range of pupil sizes.

## FIG. 1

PROVIDING AN OPTIC HAVING AN ANTERIOR AND A POSTERIOR REFRACTIVE SURFACE, WHERE THE OPTIC PROVIDES A FAR-FOCUS POWER — 1

DISPOSING A TRUNCATED DIFFRACTIVE PATTERN ON ONE OF THE SURFACES FOR GENERATING A NEAR-FOCUS ADD POWER — 2

ADJUSTING THE DIFFRACTIVE PATTERN SO AS TO OBTAIN A DESIRED DISTRIBUTION OF OPTICAL ENERGY BETWEEN THE NEAR AND FAR FOCI FOR A RANGE OF PUPIL SIZES — 3

# FIG. 2A

**Description**

**RELATED APPLICATION**

The present application is a continuation-in-part (CIP) of a U.S. patent application entitled "Apodized Aspheric Diffractive Lenses," filed on December 1, 2004, and having a Serial No. 11/000770, which is herein incorporated by reference in its entirety.

**FIELD OF THE INVENTION**

The present invention relates generally to ophthalmic lenses (e.g., intraocular lenses) and methods of correcting vision, and more particularly, to such lenses and methods that can better address the particular visual needs of individual patients and/or patient groups.

**BACKGROUND**

Intraocular lenses (IOLs) are routinely implanted in patients' eyes during cataract surgery to replace the natural crystalline lens. Some IOLs exhibit both a far-focus power as well as a near-focus power in order to provide a patient not only with far but also near vision. However, the visual needs of different patients, and/or patient groups, typically vary. For example, some patients may favor near vision over far vision, or *vice versa.* Moreover, the eyes of different patients can exhibit varying ocular parameters (e.g., different maximum pupil sizes). As a result, an IOL that provides an optimal performance for one patient may not perform as well for another patient.
Conventional IOLs and methods of their use for correcting vision, however, do not take into account such variations in patients' needs or ocular parameters.
Hence, there is a need for enhanced methods and ophthalmic lenses for correcting vision, and more particularly, for such methods and lenses that can be employed to compensate for the lost optical power of a removed natural lens.

**SUMMARY**

In one aspect, the present invention provides a method of designing a diffractive ophthalmic lens (e.g., an intraocular lens (IOL)) that includes providing an optic having an anterior refractive surface and a posterior refractive surface, wherein the optic provides a far-focus power. The far-focus optical power can be in a range of about 6 Diopters (D) to about 34 D, e.g., in a range of about 10 D to about 30 D or in a range of about 18 D to about 26 D. Further, in some cases, the far-focus optical power can be in a range of about -5 D to about 5.5 D. A truncated diffractive structure can be disposed on at least one of the surfaces for generating a near-focus add power, for example, in a range of about 2 D to about 4 D, e.g., in a range of about 2.5 D to about 4 D or in a range of about 3 D to about 4 D. And the diffractive structure can be adjusted so as to obtain a desired distribution of optical energy between the near and far foci for a range of pupil sizes. The term "truncated diffractive structure," as used herein, refers to a diffractive structure that covers a portion, rather than the entirety, of an optical surface of a lens. Further, the effective add power of the IOL when implanted in the eye can be different than its nominal (actual) add power. For example, the combination of the corneal power and the separation between the cornea and the IOL can weaken the IOL's effective add power, e.g., a nominal 4 D add power can result in a 3 D effective add power for the whole eye. In the following sections, unless otherwise indicated, the recited values of add power refer to the lens's nominal (actual) add power, which can be different that the effective add power when the IOL is implanted in the eye.
In another aspect, the diffractive structure is selected so as to obtain a desired shift in a ratio of optical energy in the far-focus relative to the energy in the near-focus as the pupil size varies over a range.
In a related aspect, adjusting the diffractive structure comprises selecting a diameter of the structure and/or the step heights of a plurality of diffractive elements forming that structure.
In another aspect, the diffractive structure can comprise a plurality of diffractive zones that exhibit apodized step heights at their boundaries. In some cases, the number of the diffractive zones can be adjusted to obtain a desired distribution of the optical energy between the near and far foci for a range of pupil sizes. Alternatively, or in addition, the variation of the step heights at the boundaries of the diffractive zones can be adjusted so as to obtain a desired energy distribution.
In another aspect, a method of designing an ophthalmic lens is disclosed that includes providing an optic that exhibits a far focus and a near focus, wherein the optic includes a diffractive structure on at least one surface thereof for generating the near focus. The diffractive structure is adjusted so as to obtain a desired distribution of optical energy between the far and near foci over a range of pupil sizes based on visual needs of a patient population. By way of example, the diffractive structure is adjusted to obtain the desired energy distribution at a design wavelength (e.g., at 550 nm).

In a related aspect, the patient population comprises patients having typical pupil diameters under photopic conditions in a range of about 2 mm to about 5 mm. In some cases, the patient population is one that favors far vision over near vision, or alternatively, favors near vision over far vision.

In another aspect, in the above method, the diffractive structure is adjusted by selecting a particular variation of step heights at boundaries of a plurality of diffractive zones, which form the structure.

In other aspects, the invention provides a method of correcting vision of a patient. The method calls for providing a lens that exhibits a far-focus power and a near focus-power for implantation in one eye of the patient, and providing another lens, which exhibits a substantially similar far-focus power but a different near-focus power, for implantation in the other eye of the patient.

In a related aspect, in the above method of correcting a patient's vision, the difference between the near-focus powers of the two lenses is selected so as to enhance the near vision range of the patient and/or to provide the patient with intermediate vision. For example, the far-focus power of each lens can be in a range of about 6 D to about 34 D while the difference between the near-focus powers of the lenses can range from about 0.2 D to about 1.5 D. For example, one lens can exhibit a near-focus power of about 4 D while the other exhibits a near-focus power of about 3 D. Alternatively, one lens can provide a near-focus power of about 4 D while the other provides a near focus power of about 3.25 or 3.75 D.

In another aspect, an ophthalmic lens is disclosed that includes an optic having an anterior surface and a posterior surface, and a diffractive structure disposed on at least one of those surfaces. The diffractive structure comprises a plurality of diffractive zones separated from one another by a plurality of steps having decreasing heights as a function of increasing radial distance from an apex of that surface. For example, the step heights can be defined in accordance with the following relation:

$$h = \frac{b * \lambda}{(n_2 - n_1)}$$

wherein

$h$ represents the physical step height,

$\lambda$ denotes the design wavelength,

$n_1$ denotes the refractive index of a medium surrounding the lens,

$n_2$ denotes the refractive index of the material forming the lens, and

$b$ is defined in accordance with the following relation:

$$b = \frac{phas0}{(1 + \dfrac{r}{rcontrol})^{rolloff}},$$

wherein

$b$ represents the phase delay as a fraction of $2\pi$ ,

*phase0* represents the overall (cumulative) optical phase delay across the diffractive steps,

$r_{control}$ represents the overall extent of the apodization region, and

*rolloff* defines the steepness of the slope of the apodization profile.

In a related aspect, in the above ophthalmic lens, the index of refraction of the material forming the lens ($n_2$) can be in a range of about 1.4 to about 1.6 (e.g., the lens can be formed of a lens material commonly known as Acrysof (a cross-linked copolymer of 2-phenylethyl acrylate and 2-phenylethyl methacrylate) having an index of refraction of 1.55). In many embodiments, the index of refraction of the surrounding medium is taken to be about 1.336. In some embodiments, the parameters *phase0, r_{contro}, rolloff* can be, respectively, in a range of about 0.4 to about 0.7, in a range of about 1 to about 2, and in a range of about 5 to about 200.

In another aspect, in the above ophthalmic lens, at least one of the anterior and posterior surfaces includes an aspheric base profile, e.g., an aspheric profile characterized by a conic constant in a range of about -10 to about -100 for the Acrysof lens material, and corresponding values can be utilized for other lens materials. In some cases, the aspheric profile can be characterized by the following relation:

$$z = \frac{cr^2}{1 + \sqrt{1 - (1+k)c^2 r^2}}$$

wherein,

$z$ denotes the surface sag at a radial location r from the apex of the surface (the intersection of the optical axis with the surface),

$c$ denotes the curvature of the surface at its apex,

$r$ denotes the radial distance from the apex of the surface, and

$k$ denotes the conic constant. In some embodiments, the parameter $c$ can range, e.g., from about 0.01 mm$^{-1}$ to about 0.1 mm$^{-1}$, while parameter $k$ (the conic constant) can range from about -10 to about-1000.

In other aspects, an ophthalmic lens is disclosed that includes an optic comprising an anterior surface and a posterior surface. The lens can further include a diffractive structure disposed on a central portion of at least one of those surfaces, where the diffractive structure is surrounded by a peripheral portion of the surface that is devoid of diffractive elements. One of the central or the peripheral portions includes an aspheric base profile while the other includes a spherical base profile. By way of example, the central portion can exhibit a spherical profile while the peripheral portion exhibits an aspherical profile characterized (e.g., by a conic constant in a range of about -10 to about -1000) or *vice versa.* In some cases, the aspheric portion can be characterized by the above relation indicating the surface sag as a function of radial distance from the optical axis.

In another aspect, an intraocular lens is disclosed that includes an anterior surface and a posterior surface, on one of which a truncated diffractive structure is disposed. The diffractive structure can be characterized by a substantially uniform step height separating adjacent diffractive elements forming the structure, or alternatively, can be characterized by apodized step heights in accordance with the above apodization function. At least one of the anterior or posterior surfaces exhibits a toric profile.

In other aspects, the above intraocular lenses can be formed from materials that provide some filtering of the blue light (e.g., wavelengths in a range of about 400 nm to about 500 nm)

Further understanding of the invention can be obtained by reference to the following detailed description, in conjunction with the associated drawings, which are described briefly below.

## BRIEF DESCRIPTION OF THE DRAWINGS

FIGURE 1 is a flow chart that depicts various steps in an exemplary embodiment of a method of the invention for designing a diffractive ophthalmic lens,

FIGURE 2A is a schematic cross-sectional view of a truncated diffractive IOL according to one embodiment of the invention,

FIGURE 2B schematically depicts a truncated diffractive structure, composed of a plurality of diffractive elements, disposed on an anterior surface of the IOL of FIGURE 2A,

FIGURE 3 is a schematic top view of the anterior surface of the IOL of FIGURE 2A, including diffractive zones disposed on that surface,

FIGURE 4 illustrates theoretically calculated curves indicating the relative energy directed to near and far foci of a plurality of hypothetical truncated diffractive IOLs having diffractive structures with different diameters,

FIGURE 5 presents a set of theoretically calculated curves depicting the distribution of optical energy between far and near foci of a plurality of truncated diffractive IOLs having different step heights at the boundaries of their diffractive zones as a function of pupil diameter,

FIGURE 6 is a schematic cross-sectional view of a diffractive IOL according to one embodiment of the invention having an apodized truncated diffractive structure on an anterior surface thereof,

FIGURE 7 presents a plurality of theoretically calculated curves illustrating the distribution of optical energy into near and far foci of a plurality of hypothetical apodized truncated diffractive lenses having diffractive structures with different diameters as a function of pupil diameter,

FIGURE 8 presents graphs illustrating theoretical distribution of energy at a design wavelength, as a function of pupil diameter, between the near and far foci of a plurality of diffractive lenses having an apodized diffractive structure in accordance with one embodiment of the invention with different apodization parameters,

FIGURE 9 is a schematic cross-sectional view of an IOL according to one embodiment of the invention having an aspherical anterior surface on which a truncated diffractive structure is disposed,

FIGURE 10 is a schematic cross-sectional view of an IOL according to another embodiment of the invention having an anterior surface on a central portion of which a truncated diffractive structure is disposed surrounded by a peripheral portion lacking diffractive structures, wherein the central portion is characterized by a spherical base profile and the peripheral portion is characterized by an aspherical base profile,

FIGURE 11 is a schematic side view of an ophthalmic lens according to one embodiment of the invention having an anterior surface with a toric base profile on which a truncated diffractive pattern is disposed.

## DETAILED DESCRIPTION OF THE PREFERRED EMBODIMENTS

The present invention generally refers to diffractive ophthalmic lenses and methods for correcting vision that employ such lenses. In the embodiments that follow, the salient features of various aspects of the invention are discussed in connection with intraocular lenses (IOLs). The teachings of the invention can also be applied to other ophthalmic lenses, such as contact lenses. The term "intraocular lens" and its abbreviation "IOL" are used herein interchangeably to describe lenses that are implanted into the interior of the eye to either replace the eye's natural lens or to otherwise augment vision regardless of whether or not the natural lens is removed. Intracorneal lenses and phakic intraocular lenses are examples of lenses that may be implanted into the eye without removal of the natural lens.

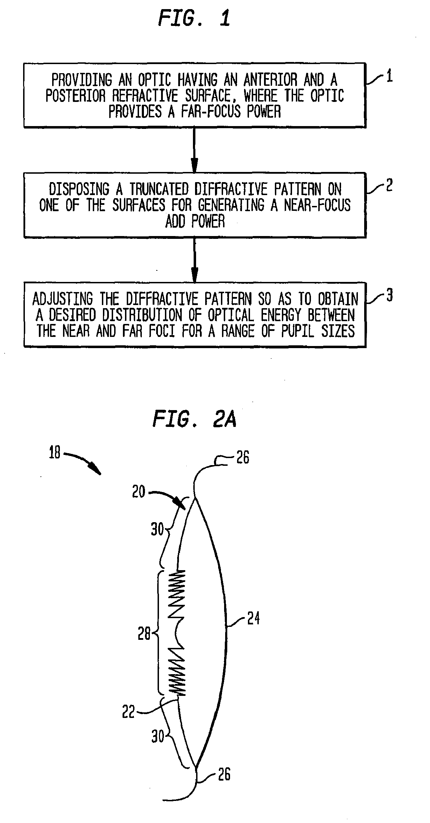

By way of example, in some embodiments, the present invention provides a method of designing an ophthalmic lens, which utilizes a diffractive structure to optimize the visual performance of the lens by adjusting the distribution of optical energy directed to a near focus and a far focus for a range of pupil sizes. With reference to a flow chart illustrated in FIGURE 1, in a method of designing a diffractive ophthalmic lens in accordance with one such embodiment, an optic having an anterior refractive surface and a posterior refractive surface is provided, where the optic provides a far-focus power (step 1). A truncated diffractive structure is disposed on one of the surfaces for generating a near-focus add power (step 2). The diffractive structure can be adjusted so as to obtain a desired distribution of optical energy between the near and far foci for a range of pupil sizes (step 3).

As discussed in more detail below, a number of parameters of the diffractive structure can be varied so as to adjust the distribution of the optical energy between the near and far foci. For example, the size of the diffractive structure, e.g., its radial extent and/or a number of diffractive zones comprising the structure, can be adjusted to obtain a desired shift in a ratio of optical energy in the far-focus relative to energy in the near-focus as the pupil size varies over a pre-defined range. As another example, in embodiments in which the diffractive structure is formed by a plurality of diffractive zones that exhibit apodized step heights at their boundaries, the variation of the step heights can be designed to obtain a desired distribution of energy between the near and far foci. A variation of the step heights is typically accompanied by changes in other parameters of the diffractive structure, such as the surface curvature within a diffractive zone.

In some embodiments, the above method can be utilized to address differences in visual needs among different patients. For example, variations of the pupil diameter (e.g., typical pupil diameter) among different patients can be addressed by providing different diffractive structures, each designed to provide a particular distribution of optical energy between the near and far foci for a given range of pupil sizes. Further, the methods of the invention can be utilized to design an ophthalmic lens that is particularly suited to the visual needs of a patient, or a group of patients. For example, for patients who favor distance vision over near vision, the diffractive structure can be selected to transmit more of the optical energy to the far focus rather than the near focus. Alternatively, the diffractive structure can be designed to emphasize near vision.

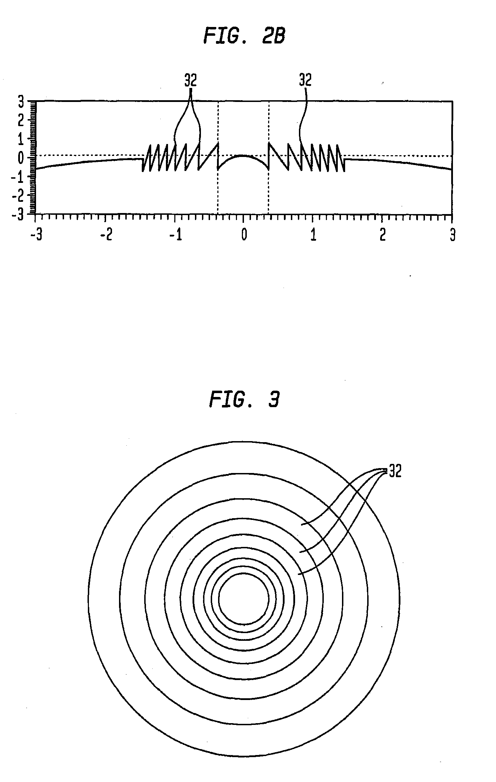

The methods of the invention can be applied to design a variety of diffractive ophthalmic lenses. By way of example, FIGURE 2A schematically depicts an intraocular lens (IOL) 18 according to one embodiment of the invention that includes an optic 20 having an anterior surface 22 and a posterior surface 24, as well as a plurality of fixation members or haptics 26 that facilitate placing the IOL in a patient's eye. Although in this embodiment, both surfaces 22 and 24 are generally convex, in other embodiments they can be concave or flat to provide other lens configurations, e.g., plano-convex. A diffractive structure 28 is disposed on a portion of the anterior surface 22, and is surrounded by a portion 30 of that surface that lacks any diffractive elements. In other words, the diffractive structure 28 is a truncated diffractive structure that covers only a portion of the surface 22. The lens 18 provides a far focus optical power, e.g., in a range of about 6 D to about 34 D (e.g., in a range of about 16 D to about 28 D), and an add power in a range of about 2 D to about 4 D, e.g., in a range of about 2.5 D to about 4 D or in a range of about 3 D to about 4 D. In other words, the near focus power can be, e.g., in a range of about 8 D to about 38 D. More particularly, the curvatures of the surfaces 22 and 24, together with the index of refraction of the optic, are configured to provide a desired far-focus optical power. The zero-diffraction order of the diffractive structure 28 transmits incident light substantially to the far focus while the first order of the diffractive structure transmits incident light to both the near and far foci. In this manner, the diffractive structure provides a desired add power, e.g., in a range of about 3 D to about 4 D. The radial diameter of the optic can range, e.g., from about 5 mm to about 7 mm, while the radial diameter of the diffractive structure can range from about 2 mm to about 5 mm. FIGURE 2B more clearly depicts a plurality of different zones 30 that form the diffraction structure. Although in this embodiment, the zeroth and 1st diffraction orders of the diffractive structure are employed for generating a near focus and a far focus, in other embodiments, other diffraction orders can be utilized for this purpose.

The lens 18 can be formed of a variety of materials, preferably biocompatible. Some examples of suitable materials include, without limitation, a soft acrylic material utilized for forming commercial lenses commonly known as Acrysof, silicone and hydrogel. By way of further examples, U.S. Patent No. 6,416,550, which is herein incorporated by reference, discloses materials suitable for forming the IOL 18.

With reference to FIGURE 2A, 2B and 3, the diffractive structures 30 form a plurality of diffractive zones 32 separated from one another at their boundaries by a substantially uniform step height, which provides a selected phase shift at each zone boundary. In some embodiments, the radial location of a zone boundary can be determined in accordance with the following relation:

$$r_i^2 = r_0^2 + 2i\lambda f \qquad \text{Eq. (1)}$$

wherein

$i$ denotes the zone number ($i = 0$ denotes the central zone)

$\lambda$ denotes the design wavelength,

$f$ denotes a focal length of the near focus, and

$r_0$ denotes the radius of the central zone

In some embodiments, the design wavelength $\lambda$ is chosen to be 550 nm green light at the center of visual response. In some cases, the radius of the central zone ($r_0$) can be set to be $\lambda f$.

Further, the step height between adjacent zones can be defined in accordance with the following relation:

$$\text{Step height} = \frac{p\lambda}{(n_2 - n_1)} \qquad \text{Eq. (2)}$$

wherein

$\lambda$ denotes the design wavelength (e.g., 550 nm),

$n_2$ denotes the refractive index of the material from which the lens is formed,

$n_1$ denotes the refractive index of the medium in which the lens is placed, and

$p$ is a fraction, e.g., 0.5 or 0.7.

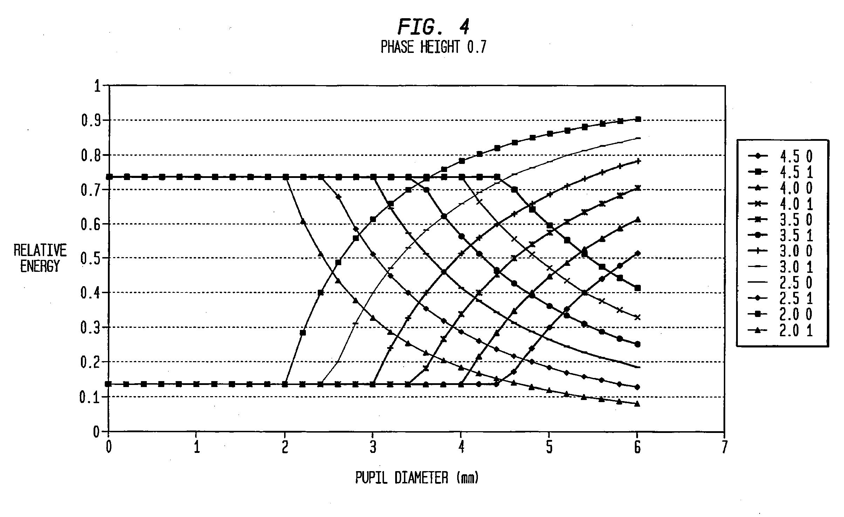

The diffractive structure 28 can be adjusted to shift the ratio of optical energy directed to the near and far foci. For example, the diameter of the diffractive structure 28 can be adjusted to vary this ratio. By way of example, FIGURE 4 illustrates a plurality of theoretically calculated curves, indicating the relative energy directed to near and far foci for a plurality of diffractive lenses, such as the above lens 18, having truncated diffractive structures. Each diffractive structure is characterized by a substantially uniform step height generating a phase delay. In this exemplary embodiment, the phase delay between adjacent zones is about 0.7$\lambda$ (where $\lambda$ was selected to be 550 nm). The diffractive structures are assumed to be disposed on a surface having a diameter of about 6 mm, with the diameter of the structures ranging from about 1.5 mm to about 4.5 mm. For this example, the step heights are selected such that for small pupil sizes, more of the energy is directed to the near focus with the ratio of the energy distributed between the near and far foci remaining substantially constant up to a threshold pupil size beyond which the energy directed to the near focus begins to decrease and the energy directed to the far focus begins to increase. These relative energy curves indicate that as the diameter of the diffractive structure increases, the threshold pupil size increases as well, which allows adjusting the distribution of the optical energy between the near and far foci. Accordingly, the diameter of the diffractive structure can be selected so as to address the visual needs of a patient, or a patient population. By way of example, for a patient who favors far vision over near vision, the diffractive structure can be selected to have a smaller diameter (e.g., a diameter of 1.5 mm).

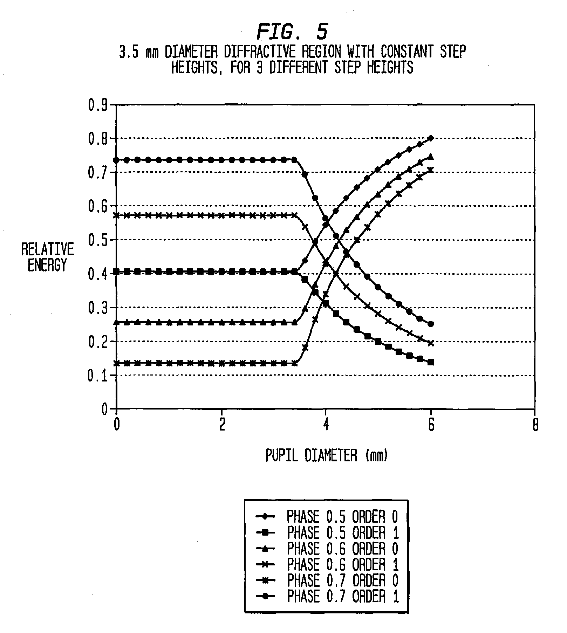

In some embodiments, the height of the step at the zone boundaries of the diffractive structure in the above diffractive lens 20 can be adjusted so as to shift the energy balance between the near and far foci. By way of example, FIGURE 5 shows a set of curves that depict the distribution of energy between far and near foci of a plurality of diffractive lenses, such as the above lens 18, having different phase delays at the zone boundaries of their respective diffractive structures. The lenses were assumed to be formed of a material used in commercial lenses known as Acrysof. The diffractive structure of each lens was assumed to have a diameter of 3.5 mm and to be formed of a plurality of zones separated from one another by steps having uniform heights for generating phase delays. The following phase delays were employed for the three lenses: 0.5 $\lambda$, 0.6 $\lambda$ and 0.7 $\lambda$ (where $\lambda$ was selected to be 550 nm). These curves indicate that the step height (phase delay) of a diffractive structure, can be adjusted (e.g., instead of or in addition to the diffractive structure's diameter) to shift the distribution of energy between the near and far foci. As noted before, in many embodiments, adjusting the step heights requires adjusting other parameters of the diffractive structures, such as the curvatures of the zones.

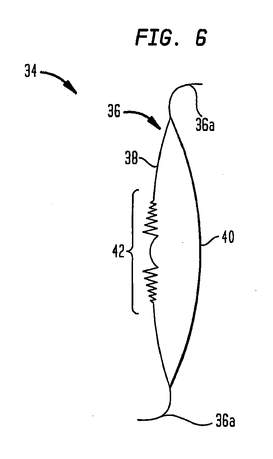

In some embodiments, the step heights at the diffractive zones vary as a function of their radial distance from the optical axis, that is, the step heights are apodized. By way of example, FIGURE 6 depicts such an embodiment of an IOL 30 having an optic 32 comprising an anterior optical surface 34 and a posterior optical surface 36. Similar to the previous embodiment, the lens 30 includes haptics 38 that facilitate its placement in the eye. A truncated apodized diffractive structure 40 is disposed on a portion of the anterior surface. The lens 30 provides a far focus optical power, for example, in a range of about 6 D to about 34 D (e.g., in a range of about 16 D to about 28 D). And the diffractive structure provides an add power in a range of about 2 D to about 4 D (e.g., in a range of about 3 D to about 4 D), so as to generate a near focus optical power (the effective add power when implanted in the eye can be somewhat different, e.g., in a range of about 1 D to about 3 D). In this exemplary embodiment, the step heights are defined in accordance with the following relation:

$$\text{Step height} = \frac{p\lambda}{n_2 - n_1} f_{apodize} \qquad \text{Eq. (3)}$$

wherein

$p$ is a phase height,

$\lambda$ is a design wavelength (e.g., 550 nm),

$n_2$ is the refractive index of the material forming the lens, and

$n_1$ is the index of refraction of the medium surrounding the lens,

$f_{apodize}$ denotes an apodization function.

A variety of apodization functions can be employed. For example, in some embodiments, the apodization function is defined in accordance with the following relation:

$$f_{apodize} = 1 - \{\frac{(r_i - r_{in})}{(r_{out} - r_{in})}\}^{exp}, \ r_{in} \le r_i \le r_{out} \qquad \text{Eq. (4)}$$

wherein

$r_i$ denotes the distance of each radial zone boundary from the intersection of the optical axis with the surface,

$r_{in}$ denotes the inner boundary of the apodization zone,

$r_{out}$ denotes the outer boundary of the apodization zone, and

$exp$ denotes an exponent to obtain a desired reduction in the step heights. Further details regarding apodization of the step heights can be found, e.g., in U.S. Patent No. 5,699,142, which is herein incorporated by reference.

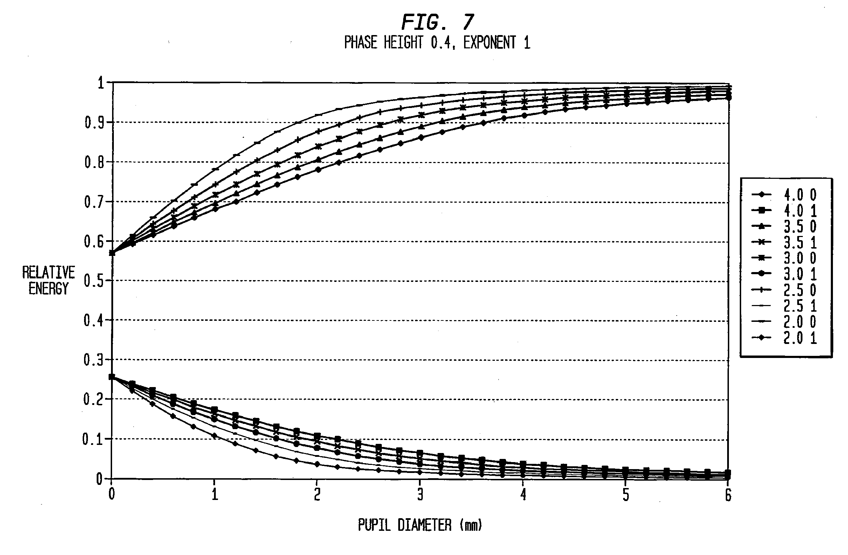

FIGURE 7 shows the distribution energy into the near and far foci of a plurality of apodized truncated diffractive lenses having diffractive structures with different diameters, whose step heights are characterized by the above relation with the following apodization parameters: phase height ($p$) = 0.4, and $exp$ = 1, as a function of the pupil size (the curves indicating the fraction of incident energy directed to the far focus are designated as corresponding to the zeroth (0) order diffraction and those indicating the fraction of energy directed to the near focus are designated as corresponding to the first (1) order diffraction).

The above energy distribution curves indicate that various parameters of an apodized diffractive structure (e.g., the diameter of the diffractive structure, phase height, and apodization exponent) can be adjusted to optimize the performance of the lens for a particular patient and/or a patient group. For example, for a patient, or a patient group, that favors near vision over far vision, a diffractive structure with a larger diameter can be selected to divert more of the incident energy to the near focus (i.e., 1st order diffraction).

In another embodiment, a different apodization of the step heights of the diffractive structure can be employed to obtain a different set of energy distribution curves indicative of the ratio of the incident light energy directed to the near and far foci. In one such embodiment, the phase delay at each diffractive step can be defined in accordance with the following relation:

$$b = \frac{phase0}{(1 + \frac{r}{rcontrol})^{rolloff}} \qquad \text{Eq. (5)}$$

wherein

$b$ represents the phase delay as a fraction of $2\pi$,

*phase0* represents the overall (cumulative) optical phase delay across the diffractive steps,

$r_{control}$ represents the overall extent of the apodization region,

*rolloff* defines the steepness of the slope of the apodization profile.

And the local diffraction efficiency as a function of radial distance from the lens center ($r$) can be determined in accordance with the following relation:

$$\text{Diffraction Efficiency} = (\frac{\sin(\alpha)}{\alpha})^2 \qquad \text{Eq. (6)}$$

wherein the parameter $\alpha$ can be defined in accordance with the following relation:

$$\alpha = \pi * (b - p) \qquad \text{Eq. (7)}$$

wherein $b$ is defined above, and $p$ denotes the diffraction order. The zeroth order diffraction is typically associated with the distance lens power.

The physical heights of the diffractive steps as a function of radial distance from the lens center can then be given by the following relation:

$$h = \frac{b * \lambda}{(n_2 - n_1)} \qquad \text{Eq. (8)}$$

wherein

$h$ represents the physical step height,

$\lambda$ denotes the design wavelength (e.g., 550 nm),

$n_1$ denotes the refractive index of a medium surrounding the lens (e.g., 1.336 ofIOLs), and

$n_2$ denotes the refractive index of the material forming the lens (e.g., 1.55 when Acrysoft is used to form the lens)

In some embodiments, the step heights follow the above relation up to a selected threshold step height (i.e., up to a certain radial location), beyond which the remaining step heights (the step heights at greater radial distances) decrease linearly to zero.

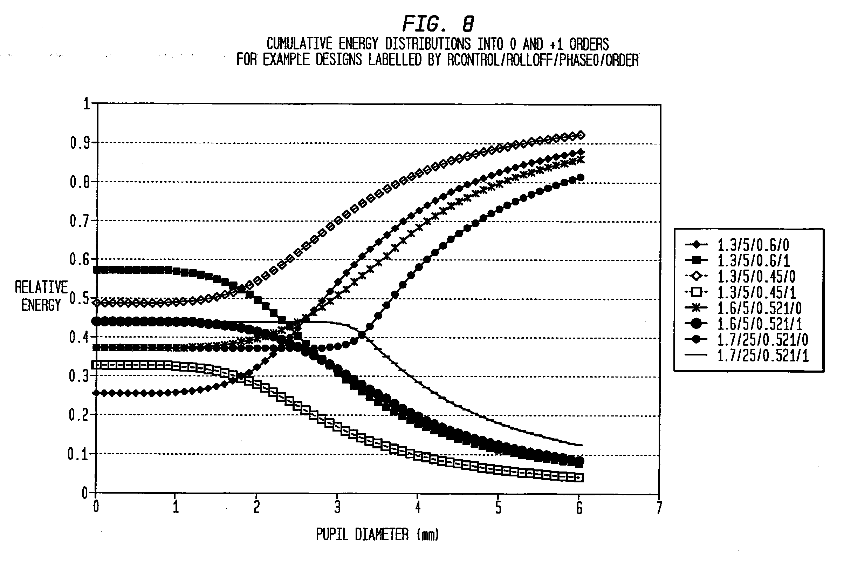

The parameters associated with the above step height apodization function (Eq. 8), such as *phase0, rolloff, rcontrol,* can be adjusted to shift the distribution of the optical energy between the near and far foci of a diffractive lens. By way of example, FIGURE 8 presents graphs illustrating theoretical distribution of energy at a design wavelength (550 nm), as a function of pupil diameter, between the near and far foci of a plurality of diffractive lenses formed of Acrysof lens material. The diffractive lenses were designed as biconvex lenses providing an add power of about 4 Diopters. Each lens includes a diffractive structure characterized by step heights that are apodized in accordance with the above apodization function (Eq. 8), albeit with apodization parameters that are different than those utilized for the other lenses. More specifically, the lenses are characterized by different values of the parameters *phase0, rolloff,* and *rcontrol.*

These graphs show that energy balance between the far-focus and the near-focus lens power as a function of the pupil size can be adjusted by varying one or more of the apodization parameters. For example, the parameters can be selected to maintain a substantially constant ratio of the optical energy directed to the near and far foci up to a selected pupil size, and to provide a ratio that varies as the pupil size grows beyond the selected value (e.g., the energy ratio would favor directing more of the light to the far focus for large pupil sizes).

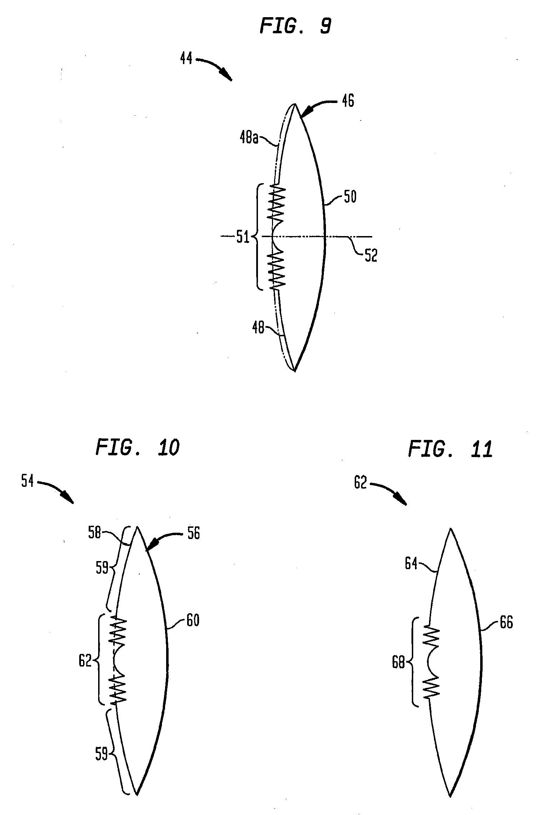

In some embodiments, one or more surfaces of the IOL can include an aspherical profile so as to reduce spherical aberration. For example, FIGURE 9 schematically depicts an IOL 42 according to such an embodiment of the invention having an optic 44 with an anterior surface 46 and a posterior surface 48. Similar to the previous embodiments, a diffractive structure 50 is far focus. By way of example, the diffractive structure can be a truncated structure having substantially uniform step heights (such as that utilized in the above lens 18) or it can include apodized step heights. In this embodiment, the anterior surface comprises an aspheric base profile, that is, a profile that is substantially coincident with a putative spherical profile 46a (shown by dashed lines) at small radial distances from an optical axis 52 but deviates from that spherical profile at greater radial distances. By way of example, the aspheric profile can be defined in accordance with the following relation:

$$z = \frac{cr^2}{1 + \sqrt{1 - (1+k)c^2 r^2}} \qquad \text{Eq. (9)}$$

wherein,

$z$ denotes the surface sag at a radial location $r$ from the apex of the surface (the intersection of the optical axis with the surface),

$c$ denotes the curvature of the surface at its apex,

$r$ denotes the radial distance from the apex of the surface, and

$k$ denotes the conic constant. By way of example, in some embodiments in which the lens is formed of Acrysof lens material, the conic constant ($k$) can be in a range of about -10 to about-1000.

FIGURE 10 schematically presents an IOL 54 in accordance with another embodiment of the invention having an optic 56 with an anterior surface 58 and a posterior surface 60. Similar to the previous embodiments, a diffractive structure 62, characterized by substantially uniform or apodized step heights, is disposed on a portion of the anterior surface 58. A peripheral portion 59 of the anterior surface, i.e., a portion of the surface surrounding the diffractive structure, is characterized by a substantially spherical profile. In contrast, the central portion of the anterior surface, i.e., a portion of the surface on which the diffractive structure is disposed, is characterized by an aspherical profile (shown by dashed lines), e.g., a profile defined by the above Eq. (9). Alternatively, in other embodiments, the central portion is characterized by a spherical profile while the peripheral portion exhibits a selected degree of asphericity.

In other aspects, the present invention discloses methods for correcting vision that can enhance the range of a patient's near vision and/or to provide a patient with not only far and near vision but also an intermediate vision. For example, one diffractive IOL having one add power can be implanted in one eye of a patient and another diffractive IOL having a different add power can be implanted in the other eye. The difference in the add powers can be, e.g., in a range of about 0.1 D to about 1.5 D (e.g., in a range of about 0.2 to 1 D). For example, a combination of the following add powers can be employed to enhance a patient's range of near vision: 4 D, 3.75 D, 3.5 D, 3.25 D and 3 D. In some embodiments, two diffractive IOLs having substantially similar far focus powers but different add powers can be implanted in a patient's eyes (each in one eye of the patient) such that the IOL with the lower add power would provide a degree of intermediate vision and/or enhance the range of the near vision. In some embodiments, the difference between the add powers of the two lenses can be selected such that their near vision energy curves would at least partially overlap so as to enhance the depth of focus for near vision.

The above method of implanting a different IOL in each eye of a patient can be combined with the previous methods of optimizing the distribution of energy between the near and far foci so as to enhance a patient's vision. For example, for a patient favoring near and intermediate vision over far vision, the diffractive structures of the lenses can be adjusted, in a manner discussed above, so as to optimize the incident light energy that is directed to the near focus over a range of pupil sizes.

In other embodiments, one or both optical surfaces of a truncated diffractive lens can exhibit a selected degree of toricity to provide, e.g., astigmatic correction. By way of example, FIGURE 11 schematically illustrates a diffractive lens 62 according to such an embodiment of the invention that includes an anterior optical surface 64 and a posterior optical surface 66. A truncated diffractive structure 68 is disposed on the anterior surface. In this embodiment, the diffractive structure is characterized by a plurality of diffractive zones separated from one another by substantially uniform step heights (in other embodiments, a diffractive structure with apodized step heights, e.g., a structure with step heights defined by the above Eq. (8), can be utilized). The anterior surface 64 includes a toric profile characterized by different curvatures in two orthogonal directions.

In some embodiments, the diffractive IOL (having a truncated diffractive structure characterized by a substantially uniform or a non-uniform step heights) can be formed of a material that can provide some filtering of the blue light. By way of example, the IOL can be formed of Acrysof Natural material. By way of further example, U.S. Patent No. 5,470,932, herein incorporated by reference, discloses polymerizable yellow dyes that can be utilized to block or lower the intensity of blue light transmitted through ocular lenses.

The various lenses discussed above can be formed by employing manufacturing techniques known in the art.

Those having ordinary skill in the art will appreciate that various modifications can be made to the above embodiments without departing from the scope of the invention.

**Claims**

1.  A method of designing a diffractive ophthalmic lens, comprising

    providing an optic having an anterior refractive surface and a posterior refractive surface, said optic providing a far-focus power,
    disposing a truncated diffractive structure on one of said surfaces for generating a near-focus add power,
    adjusting the diffractive structure so as to obtain a desired distribution of optical energy between said near and far foci for a range of pupil sizes.

2.  The method of claim 1, wherein the step of adjusting the diffractive structure comprises selecting a diameter of said structure.

3.  The method of claim 1, wherein said diffractive structure comprises a plurality of diffractive zones exhibiting apodized step heights at boundaries thereof.

4.  The method of claim 3, wherein the step of adjusting the diffractive structure comprises selecting a number of the diffractive zones.

5.  The method of claim 3, wherein the step of adjusting the diffractive structure comprises selecting a variation of the step heights at the zone boundaries.

6.  The method of claim 1, wherein said far-focus power is in a range of about 16 D Diopters to about 28 Diopters.

7.  The method of claim 5, wherein said near-focus add power is in a range of about 3 Diopters to about 4 Diopters.

8.  The method of claim 1, wherein the step of adjusting the diffractive structure comprises selecting the diffractive structure so as to obtain a desired shift in a ratio of optical energy in the far-focus relative to energy in the near-focus as the pupil size varies over a pre-defined range.

9.  The method of claim 1, wherein said ophthalmic lens comprises an intraocular lens.

10. The method of claim 1, wherein said ophthalmic lens comprises a contact lens.

11. A method of designing an ophthalmic lens, comprising

    providing an optic exhibiting a far focus and a near focus, said optic having a diffractive structure on at least one surface thereof for generating the near focus, and
    adjusting the diffractive structure so as to obtain a desired distribution of optical energy between said far and near foci over a range of pupil sizes based on visual needs of a patient population.

12. The method of claim 11, further comprising adjusting the diffractive structure to obtain the desired energy distribution at a design wavelength.

13. The method of claim 12, wherein said design wavelength is selected to be about 550 nm.

14. The method of claim 11, wherein said patient population comprises patients having pupil diameters in a range of about 2 mm to about 5 mm under photopic conditions.

**15.** The method of claim 11, wherein said patient population favors far vision over near vision.

**16.** The method of claim 11, wherein said patient population favors near vision over far vision.

**17.** The method of claim 11, wherein adjusting the diffractive structure comprises selecting a number of diffractive zones comprising that structure.

**18.** The method of claim 11, wherein adjusting the diffractive structure comprises selecting a variation of step heights at boundaries of a plurality of diffractive zones comprising the diffractive structure.

**19.** The method of claim 11, wherein adjusting the diffractive structure comprises selecting a phase delay generated by the structure at a center thereof.

**20.** A method of correcting vision of a patient, comprising

providing an optic exhibiting a far-focus power and a near focus power for correcting vision in one eye of the patient,
providing another optic exhibiting a far-focus power and a near focus power for correcting vision in the other eye of the patient,

wherein said optics have substantially similar far-focus power and different near-focus power.

**21.** The method of claim 20, further comprising selecting said far-focus power to be in a range of about 16 Diopters to about 28 Diopters.

**22.** The method of claim 21, wherein each optic provides a near-focus add power in a range of about 2.5 to about 4 Diopters.

**23.** The method of claim 22, wherein one optic provides a near-focus add power of about 4 D and the other optic provides a near-focus add power of about 3 D.

**24.** The method of claim 22, wherein one optic provides a near-focus add power of about 4 D and the other optic provides a near-focus add power of about 3.25 D.

**25.** The method of claim 22, wherein one optic provides a near-focus add power of about 4 D and the other optic provides a near-focus add power of about 3.5 D.

**26.** The method of claim 22, wherein one optic provides a near-focus add power of about 4 D and the other optic provides a near-focus add power of about 3.75 D.

**27.** The method of claim 20, wherein each optic comprises a diffractive structure disposed on a surface thereof for generating said far and near focus power.

**28.** An ophthalmic lens, comprising
an optic having an anterior surface and a posterior surface,
a diffractive structure disposed on at least one of said surfaces,
said diffractive structure comprising a plurality of diffractive zones separated from one another by a plurality of steps having decreasing heights as a function of radial distance from an apex of said surface,
wherein said step heights are defined in accordance with the following relation:

$$h = \frac{b * \lambda}{(n_2 - n_1)}$$

wherein
$h$ represents the physical step height,

λ denotes the design wavelength,
$n_1$ denotes the refractive index of a medium surrounding the lens,
$n_2$ denotes the refractive index of the material forming the lens, and
$b$ is defined in accordance with the following relation:

$$b = \frac{phas0}{(1 + \frac{r}{rcontrol})^{rolloff}},$$

wherein
$b$ represents the phase delay as a fraction of $2\pi$,
$phase0$ represents the overall (cumulative) optical phase delay across the diffractive steps,
$r_{control}$ represents the overall extend of the apodization region,
$rolloff$ defines the steepness of the slope of the apodization profile.

29. The ophthalmic lens of claim 28, wherein
$Phase0$ can be in a range of about 0.4 to about 0.7,
$r_{control}$ can be in a range of about 1 to about 2, and
$rolloff$ can be in a range of about 5 to about 200.

30. The ophthalmic lens of claim 28, wherein at least one of said anterior or posterior surfaces includes an aspherical base profile.

31. The ophthalmic lens of claim 30, wherein said profile is **characterized by** a conic constant in a range of about -10 to about -1000.

32. The ophthalmic lens of claim 30, wherein said base profile is defined by the following relation:

$$z = \frac{cr^2}{1 + \sqrt{1 - (1+k)c^2 r^2}}$$

wherein,
$z$ denotes the surface sag at a radial location r from the apex of the surface (the intersection of the optical axis with the surface),
$c$ denotes the curvature of the surface at its apex,
$r$ denotes the radial distance from the apex of the surface, and
$k$ denotes the conic constant,
wherein,
$c$ can be in range of about 0.001 1 mm$^{-1}$ to about 0.1 mm$^{-1}$,
$r$ can range from about 0 to about 7 mm, and
$k$ can be in a range of about -10 to about -1000.

33. The ophthalmic lens of claim 28, wherein at least one of said anterior or posterior surfaces exhibits a toric base profile.

34. An ophthalmic lens, comprising
an optic comprising an anterior surface and a posterior surface,
a diffractive structure disposed on a central portion of one of said surfaces surrounded by a peripheral portion of the surface that is devoid of diffractive structures,
wherein one of said peripheral or central portions is **characterized by** a spherical base profile and the other portion is **characterized by** an aspherical base profile.

35. The ophthalmic lens of claim 34, wherein said ophthalmic lens comprises an IOL.

**36.** An ophthalmic lens, comprising
an optic having an anterior surface and a posterior surface, and
a truncated diffractive structure disposed on a portion of one of said surfaces, said diffractive structure being **characterized by** a plurality of diffractive zones separated from one another by substantially uniform step heights, wherein at least one of said surfaces exhibits a toric base profile.

**37.** The ophthalmic lens of claim 36, wherein said ophthalmic lens comprises an IOL.

## FIG. 1

PROVIDING AN OPTIC HAVING AN ANTERIOR AND A
POSTERIOR REFRACTIVE SURFACE, WHERE THE OPTIC
PROVIDES A FAR-FOCUS POWER  ⌐1

DISPOSING A TRUNCATED DIFFRACTIVE PATTERN ON
ONE OF THE SURFACES FOR GENERATING A NEAR-FOCUS
ADD POWER  ⌐2

ADJUSTING THE DIFFRACTIVE PATTERN SO AS TO OBTAIN
A DESIRED DISTRIBUTION OF OPTICAL ENERGY BETWEEN
THE NEAR AND FAR FOCI FOR A RANGE OF PUPIL SIZES  ⌐3

## FIG. 2A

## FIG. 2B

## FIG. 3

*FIG. 4*
PHASE HEIGHT 0.7

RELATIVE
ENERGY

PUPIL DIAMETER (mm)

4.5 0
4.5 1
4.0 0
4.0 1
3.5 0
3.5 1
3.0 0
3.0 1
2.5 1
2.0 0
2.0 1

## FIG. 5
### 3.5 mm DIAMETER DIFFRACTIVE REGION WITH CONSTANT STEP HEIGHTS, FOR 3 DIFFERENT STEP HEIGHTS

RELATIVE ENERGY

PUPIL DIAMETER (mm)

—♦— PHASE 0.5 ORDER 0
—■— PHASE 0.5 ORDER 1
—▲— PHASE 0.6 ORDER 0
—✕— PHASE 0.6 ORDER 1
—✳— PHASE 0.7 ORDER 0
—●— PHASE 0.7 ORDER 1

## FIG. 6

## FIG. 7
### PHASE HEIGHT 0.4, EXPONENT 1

RELATIVE ENERGY (y-axis), PUPIL DIAMETER (mm) (x-axis)

Legend:
- 4.0 0
- 4.0 1
- 3.5 0
- 3.5 1
- 3.0 0
- 3.0 1
- 2.5 0
- 2.5 1
- 2.0 0
- 2.0 1

EP 1 891 912 A1

FIG. 8

CUMULATIVE ENERGY DISTRIBUTIONS INTO 0 AND +1 ORDERS
FOR EXAMPLE DESIGNS LABELLED BY RCONTROL/ROLLOFF/PHASE0/ORDER

FIG. 9

FIG. 10

FIG. 11

**European Patent**
**Office**

**PARTIAL EUROPEAN SEARCH REPORT**

which under Rule 45 of the European Patent Convention
shall be considered, for the purposes of subsequent
proceedings, as the European search report

**Application Number**

EP 07 11 4347

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | US 2006/116764 A1 (SIMPSON MICHAEL J [US]) 1 June 2006 (2006-06-01) * the whole document * | 1-19, 34-37 | INV. A61F2/16 G02C7/04 |
| D,X | US 5 699 142 A (LEE CHUN-SHEN [US] ET AL) 16 December 1997 (1997-12-16) * the whole document * | 1-5,7-19 | |
| A | | 6,34-37 | |
| D,A | US 6 416 550 B2 (FREEMAN CHARLES [US]) 9 July 2002 (2002-07-09) * the whole document * | 1-19, 34-37 | |
| D,A | US 5 470 932 A (JINKERSON DAVID L [US]) 28 November 1995 (1995-11-28) * the whole document * | 1-19, 34-37 | |

| TECHNICAL FIELDS SEARCHED (IPC) |
|---|
| A61F G02C |

### INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 24 September 2007 | Geuer, Melanie |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C07)

Claim(s) searched incompletely:
1-19, 34-37

Claim(s) not searched:
20-33

Reason for the limitation of the search (non-patentable invention(s)):

Claim 20 is a methods for treatment of the human or animal body by surgery, it comprises the correction of the vision of a patient in one eye of the patien, which involves a surgical intervention. Hence claim 20 and claims 21-27 which are dependent from claim 20 must be deleted, Article 52 (4) EPC - Method for treatment of the human or animal body by surgery
Claim 28 and dependent claims 29-33 relate to a product defined (inter alia) by reference unusual formulas. The use of this unusual formulas in the present context is considered to lead to a lack of clarity because the claim does not clearly identify the products encompassed by it as the formulas cannot be clearly and reliably determined by indications in the description or by objective procedures which are usual in the art. This makes it impossible to compare the claim to the prior art, Rule 45 EPC.

**ANNEX TO THE EUROPEAN SEARCH REPORT
ON EUROPEAN PATENT APPLICATION NO.**

EP 07 11 4347

This annex lists the patent family members relating to the patent documents cited in the above-mentioned European search report.
The members are as contained in the European Patent Office EDP file on
The European Patent Office is in no way liable for these particulars which are merely given for the purpose of information.

24-09-2007

| Patent document cited in search report | | Publication date | Patent family member(s) | | Publication date |
|---|---|---|---|---|---|
| US 2006116764 | A1 | 01-06-2006 | AU 2005311949 A1<br>CA 2590085 A1<br>US 2007171362 A1<br>WO 2006060480 A2 | | 08-06-2006<br>08-06-2006<br>26-07-2007<br>08-06-2006 |
| US 5699142 | A | 16-12-1997 | NONE | | |
| US 6416550 | B2 | 09-07-2002 | US 2001000351 A1 | | 19-04-2001 |
| US 5470932 | A | 28-11-1995 | AT 176268 T<br>AU 674262 B2<br>AU 8014794 A<br>CA 2147856 A1<br>DE 69416293 D1<br>DE 69416293 T2<br>DK 674684 T3<br>EP 0674684 A1<br>ES 2127419 T3<br>GR 3029577 T3<br>HK 1013092 A1<br>JP 3375841 B2<br>JP 9187499 A<br>JP 3375842 B2<br>JP 9187500 A<br>JP 2617097 B2<br>JP 8503997 T<br>JP 2003119226 A<br>WO 9511279 A1<br>US 5543504 A<br>US 5528322 A | | 15-02-1999<br>12-12-1996<br>08-05-1995<br>27-04-1995<br>11-03-1999<br>02-06-1999<br>13-09-1999<br>04-10-1995<br>16-04-1999<br>30-06-1999<br>07-04-2000<br>10-02-2003<br>22-07-1997<br>10-02-2003<br>22-07-1997<br>04-06-1997<br>30-04-1996<br>23-04-2003<br>27-04-1995<br>06-08-1996<br>18-06-1996 |

EPO FORM P0459

For more details about this annex : see Official Journal of the European Patent Office, No. 12/82

**EP 1 891 912 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11000770 B
- US 6416550 B
- US 5699142 A
- US 5470932 A